# EUROPÄISCHE PATENTSCHRIFT

(11) **EP 1 699 747 B1**
(45) Veröffentlichungstag und Bekanntmachung des Hinweises auf die Patenterteilung: **21.05.2008**
(21) Anmeldenummer: 04803863.2
(22) Anmeldetag: 14.12.2004
(51) Int. Cl.: C07C 45/74

(54) **VERFAHREN ZUR HERSTELLUNG VON 2,3-CIS-SUBSTITUIERTEN 2-ARYLPROPENALEN**
METHOD FOR THE PRODUCTION OF 2,3-CIS-SUBSTITUTED 2-ARYL PROPENALS
PROCEDE POUR LA PRODUCTION DE 2-ARYLPROPENALS 2,3-CIS-SUBSTITUES

(30) Priorität: 15.12.2003 DE 10358655
(43) Veröffentlichungstag der Anmeldung: 13.09.2006
(73) Patentinhaber: BASF SE, 67056 Ludwigshafen (DE)
(72) Erfinder: NOACK, Reiner, 04932 Grossthiemig (DE); RÜB, Lothar, 67346 Speyer (DE); PALM, Clemens, 01109 Dresden (DE)
(74) Vertreter: Reitstötter - Kinzebach
(86) Internationale Anmeldenummer: PCT/EP2004/014241
(87) Internationale Veröffentlichungsnummer: WO 2005/056498

(56) Entgegenhaltungen:
- EP-A- 0 352 675
- DATABASE CROSSFIRE BEILSTEIN [Online] Beilstein Institut für Förderung der Chemischen Wissenschaften, Frankfurt am main DE; 27. Juni 1988 (1988-06-27), XP002335449 Database accession no. 573006 (REACTION ID) & SCHORIGIN ET AL: CHEM. BER., Bd. 66, 1933, Seiten 389-393, XP009050389
- COURTOT P., AUFFRET J.: J.CHEM.RES.MINIPRINT, Bd. 10, 1981, Seiten 3516-3528, XP009050380
- CLIMENT,MJ.ET AL: "Aldol Condensation on Solid catalysts: A Cooperative Effect between Weak Acid and Base Sites" ADV.SYNTH.CATAL., Bd. 344, Nr. 10, 2002, Seiten 1090-1096, XP002335364
- JI-HYUN KIM AND KULAWIEC R.J: "A Tandem Epoxide Isomerization-Aldol Condensation Process Catalyzed by Palladium Acetate Tributylphosphine" J.ORG.CHEM, Bd. 61, 1996, Seiten 7656-7657, XP002335226
- STERN R.L., KRAUSE,J.G.: "Enazine Chemistry II: The Mechanism of formation of 1-(cis-3-Phenylpropenyl)- 5-Phenylpyrazole from the Pyrolysis of Cinnamaldehyde Azine" J.HETEROCYCL.CHEM, Bd. 5, 1968, Seiten 263-268, XP002335227

## Beschreibung

Die folgende Erfindung betrifft ein Verfahren zur Herstellung von 2,3-*cis*-substituierten 2-Arylpropenalen durch Kondensation eines 2-Arylacetaldehyds I mit einer nicht-enolisierbaren Aldehydverbindung II in Gegenwart einer Base.

2,3-*cis*-substituierte 2-Arylpropenale, insbesondere die in 3-Stellung mit einer weiteren Arylgruppe substituierten Diphenylpropenale, dienen zur Herstellung von Hydroxymethyloxiranen, die ihrerseits bei der Synthese von fungiziden Wirkstoffen benötigt werden.

Für die Herstellung derart substituierter Propenale sind verschiedene Verfahren bekannt, bei denen in der Regel der Einsatz spezieller Katalysatorsysteme erforderlich ist.

Es ist bekannt, dass 2,3-substituierte Propenale mittels einer gekreuzten Aldolkondensation hergestellt werden können. Derartige Reaktionen werden im Allgemeinen in einem organischen Lösungsmittel oder gegebenenfalls, bei Verwendung geeigneter Edukte, in Substanz durchgeführt. In Abhängigkeit von Struktur und Reaktivität der eingesetzten Aldehyde werden dabei jedoch neben den gewünschten Produkten mit *cis*-Konfiguration häufig erhebliche Anteile von Nebenprodukten erhalten (siehe z. B. Houben-Weyl, Methoden der organischen Chemie, Band VII/1, Georg-Thieme-Verlag, Stuttgart, 1954, 76ff; Alder et al., Ann. Chem. 586, 1954, 110).

Eine weitere Schwierigkeit besteht in oft niedrigen Gesamtausbeuten der Reaktion. Diese sind vermutlich Folge der hohen Empfindlichkeit der Reaktionsprodukte gegenüber Säuren und Basen. So beschreibt die DE 38 25 586 die Herstellung von substituierten 2,3-Bis(phenyl)propenalen wie E-2-(4-fluorphenyl)-3-(2-trifluormethylphenyl)-propenal mittels gekreuzter Aldolkondensation mit einer Ausbeute von lediglich 65%. Die geschilderten Schwierigkeiten gelten analog auch bei der DE 37 22 886, die die Reaktion kernsubstituierter Phenylacetaldehyde mit einer weiteren Aldehydverbindung beschreibt. Die Frage der Stereoselektivität wird dort nicht angesprochen. Die Reaktion erfolgt dabei in einem organischen Lösungsmittel.

Im Fall der 2-Phenylalkanale wurde die Natur der entstehenden Nebenprodukte genauer untersucht. Dabei wurde eine Neigung zur Selbstkondensation unter Bildung dimerer und trimerer Oligomerer bzw. gemischter Trimerer festgestellt. Im Einzelnen wurden von Treibs et al., Chem. Ber. 85, 1952, 1116, Triphenylpyranderivate und deren Folgeprodukte; sowie von Ericksen et al., J. Am. Chem. Soc. 80, 1958, 5466, Aldoxane und deren Folgeprodukte als spezielle Trimere identifiziert.

Die Bildung von Nebenprodukten, wie beschrieben, führt zu einer Minimierung der Ausbeute an den gewünschten 2,3-*cis*-substituierten 2-Arylpropenalen. In der Regel führt die Anwesenheit unerwünschter Nebenprodukte zu erheblichen Schwierigkeiten bei der Aufarbeitung der Wertprodukte. Es besteht daher ein Bedarf nach einem wirtschaftlichen Verfahren zur Herstellung von 2,3-*cis*-substituierten 2-Arylpropenalen.

Der vorliegenden Erfindung lag somit die Aufgabe zu Grunde, ein Verfahren zur Herstellung von 2,3-*cis*-substituierten 2-Arylpropenalen bereitzustellen, das die Nachteile des Stands der Technik vermeidet. Das Verfahren sollte insbesondere die gewünschten 2,3-*cis*-substituierten 2-Arylpropenale mit hohen Ausbeuten und gleichzeitig hoher Stereoselektivität liefern. Weiterhin sollte es die Bildung von Nebenprodukten weitgehend vermeiden sowie eine technisch einfache Durchführung gewährleisten.

Diese Aufgabe wurde überraschend gelöst, indem man einen 2-Arylacetaldehyd I mit einer nicht-enolisierbaren Aldehydverbindung II in Gegenwart einer Base in einem Lösungsmittelgemisch umsetzt, das wenigstens ein mit Wasser mischbares organisches Lösungsmittel und Wasser in einem vorgegebenen Volumenverhältnis umfasst.

Somit betrifft die vorliegende Erfindung ein Verfahren zur Herstellung von 2,3-*cis-*substituierten 2-Arylpropenalen durch Kondensation eines 2-Arylacetaldehyds I mit einer nicht-enolisierbaren Aldehydverbindung II in Gegenwart einer Base, dadurch gekennzeichnet, dass man die Umsetzung in einem Lösungsmittelgemisch durchführt, das wenigstens ein mit Wasser mischbares organisches Lösungsmittel.und Wasser in einem Volumenverhältnis V_{Lösungsmittel} : V_{Wasser} im Bereich von 10:1 bis 0,5:1 und insbesondere von 5:1 bis 0,5:1 umfasst, wobei man den 2-Arylacetaldehyd I und die nicht-enolisierbare Aldehydverbindung II in einem molaren Verhältnis von I:II im Bereich von 1:1,05 bis 1:1:5 einsetzt.

Der Ausdruck "2,3-*cis*-substituierte 2-Arylpropenale" bezeichnet hier und im Folgenden eine *cis*-Konfiguration der erfindungsgemäßen 2-Arylpropenale bezüglich der Arylgruppe in 2-Position und einem weiteren von Wasserstoff verschiedenen Substituenten in 3-Position. In synonymer Weise ist auch der hier und im Folgenden verwendete Ausdruck "*cis*-Konfiguration" zu verstehen. Bezüglich der Stellung von Aldehydgruppe und Substituent in der 3-Position handelt es sich um die E-Isomere.

Die Arylgruppe der erfindungsgemäß eingesetzten 2-Arylacetaldehyde I umfasst üblicherweise einen gegebenenfalls substituierten aromatischen Kohlenwasserstoffrest, wie Phenyl, Naphthyl oder Anthryl, insbesondere Phenyl.

Des Weiteren kann die Arylgruppe der erfindungsgemäß eingesetzten 2-Arylacetaldehyde I einen oder mehrere, z. B. 1, 2, 3, 4 oder 5, Substituenten R' aufweisen, die unabhängig voneinander unter
- C₁-C₁₀-Alkyl, das gegebenenfalls einen oder mehrere von Alkyl verschiedene Substituenten, die unter Fluor, Chlor, Brom, Iod, C₁-C₁₀-Alkoxy oder C₃-C₁₀-Cycloalkyl ausgewählt sein können, aufweist;
- C₁-C₁₀-Alkoxy, das gegebenenfalls einen oder mehrere von Alkyl verschiedene Substituenten, die unter Fluor, Chlor, Brom, Iod, C₁-C₁₀-Alkoxy oder C₃-C₁₀-Cycloalkyl ausgewählt sein können, aufweist;
- C₃-C₁₀-Cycloalkyl, das gegebenenfalls einen oder mehrere von Alkyl verschiedene Substituenten, die unter Fluor, Chlor, Brom, Iod oder C₁-C₁₀-Alkoxy ausgewählt sein können, aufweist;
- Phenoxy, das gegebenenfalls durch 1, 2, 3, 4 oder 5 unter C₁-C₄-Alkyl, C₁-C₄-Alkoxy, Fluor, Chlor, Brom oder Iod ausgewählte Gruppen substituiert ist;
- Halogen, das unter Fluor, Chlor, Brom oder Iod ausgewählt ist;
- C₁-C₁₀-Acylamino; oder
- einer Nitrogruppe
ausgewählt sind.

Hier und im Folgenden steht Alkyl für einen linearen oder verzweigten aliphatischen Kohlenwasserstoffrest mit in der Regel 1 bis 10, insbesondere 1 bis 6 und speziell 1 bis 4 C-Atomen, wie z. B. Methyl, Ethyl, n-Propyl, 1-Methylethyl, n-Butyl, 1-Methylpropyl, 2-Methylpropyl oder 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyt, 1-Methylpentyl, 2-Methylpentyl, 3-Methylpentyl, 4-Methylpentyl, 1,1-Dimethylbutyl, 1,2-Kimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl, 1-Ethyl-3-methylpropyl, n-Heptyl, n-Octyl, n-Nonyl, n-Decyl, 1-Methylhexyl, 1-Ethylhexyl, 2-Ethylhexyl, 1-Methylheptyl, 1-Methyloctyl, 1-Methylnonyl. Die genannten Alkylreste können gegebenenfalls einen oder mehrere, z. B. 1, 2, 3 oder 4, von Alkyl verschiedene Substituenten aufweisen, die unter Halogen, wie z. B. Fluor, Chlor, Brom oder Iod, C₁-C₁₀-Alkoxy, wie in C₁-C₁₀-Alkoxy-C₁-C₁₀-Alkyl, z. B. 2-Methoxyethyl, oder C₃-C₁₀-Cycloalkyl, wie in C₃-C₁₀-Cycloalkyl-C₁-C₁₀-Alkyl, z. B. Cyclohexylmethyl, ausgewählt sein können.

Hier und im Folgenden steht Halogen für ein unter Fluor, Chlor, Brom oder Iod ausgewähltes Halogenatom.

Hier und im Folgenden steht Alkoxy für einen über ein Sauerstoffatom gebundenen Alkylrest, wie oben definiert, mit in der Regel 1 bis 10, insbesondere 1 bis 6 und speziell 1 bis 4 C-Atomen, wie z. B. Methoxy, Ethoxy, Propoxy, 1-Methylethoxy, Butoxy, 1-Methylpropoxy, 2-Methylpropoxy oder 1,1-Dimethylethoxy. Die genannten Alkoxyreste können gegebenenfalls einen oder mehrere, z. B. 1, 2, 3 oder 4, von Alkyl verschiedene Substituenten aufweisen, die unter Halogen, wie z. B. Fluor, Chlor, Brom oder Iod, C₁-C₁₀-Alkoxy, wie in C₁-C₁₀-Alkoxy-C₁-C₁₀-Alkoxy, z. B. 2-Methoxyethoxy, oder C₃-C₁₀-Cycloalkyl, wie in C₃-C₁₀-Cycloalkyl-C₁-C₁₀-Alkoxy, z. B. Cyclohexylmethoxy, ausgewählt sein können.

Hier und im Folgenden steht Cycloalkyl für einen cycloaliphatischen Rest mit in der Regel 3 bis 10, insbesondere 3 bis 6 und speziell 3 bis 4 C-Atomen, wie z. B. Cyclopropyl, Cyclobutyl, Cyclopentyl, Cyclohexyl, Cycloheptyl, Cyclooctyl, Cyclononyl oder Cyclodecyl. Die genannten Cycloalkylreste können gegebenenfalls einen oder mehrere, z. B. 1, 2, 3 oder 4, von Alkyl verschiedene Substituenten aufweisen, die unter Halogen, wie z. B. Fluor, Chlor, Brom oder Iod, oder C₁-C₁₀-Alkoxy, wie z.B. Ci-C₆-Alkoxy oder C₁-C₄-Alkoxy, ausgewählt sein können. Beispiele für substituiertes Cycloalkyl sind insbesondere 4-Methylcyclohexyl, 3,3,5,5-Tetramethylcyclohexyl und dergleichen.

Hier und im Folgenden steht C₁-C₁₀-Acylamino für einen über eine Aminogruppe NR"', worin R"' für Wasser-stoff oder C₁-C₆-Alkyl steht, gebundenen Alkanoylrest mit einem linearen oder verzweigten aliphatischen Kohlenwasserstoffrest mit in der Regel 1 bis 10, insbesondere 1 bis 6 und speziell 1 bis 4 C-Atomen, wie z. B. Methanoylamino, Ethanoylamino, n-Propanoylamino, iso-Propanoylamino, n-Butanoylamino, iso-Butanoylamino und tert-Butanoylamino.

In einer bevorzugten Ausführungsform handelt es sich bei den 2-Arylacetaldehyden 1 um 2-Phenylacetaldehyde, deren Phenylgruppe gegebenenfalls einen oder mehrere, z.B. 1, 2, 3, 4 oder 5, unabhängig voneinander unter R', wie oben definiert, ausgewählte Substituenten aufweist.

Derartige Verbindungen lassen sich durch die folgende Formel la beschreiben 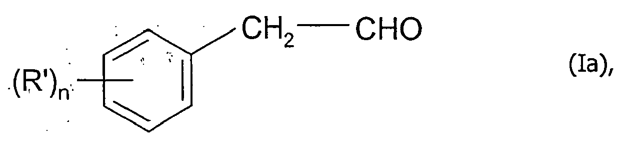 worin R' für n > 1 gleich oder verschieden sein kann und die zuvor genannten Bedeutungen aufweist; n für 0, 1, 2, 3, 4 oder 5, insbesondere für 0, 1 oder 2 und speziell für 1 steht. R' ist insbesondere ausgewählt unter Halogen.

In einer besonders bevorzugten Ausführungsform steht in der obigen Formel Ia R' für Halogen und n für 1. R' ist dann insbesondere in der 4-Position angeordnet.

In einer weiteren besonders bevorzugten Ausführungsform wird als 2-Arylacetaldehyd I ein Phenylacetaldehyd eingesetzt. Der eingesetzte Phenylacetaldehyd weist am Phenylring gegebenenfalls einen oder mehrere unter Fluor, Chlor, Brom oder Iod ausgewählte Substituenten auf. Ganz besonders bevorzugt wird 4-Fluorphenylacetaldehyd eingesetzt.

Die erfindungsgemäß eingesetzten nicht-enolisierbaren Aldehydverbindungen II zeichnen sich dadurch aus, dass das C-Atom in α-Position zur Aldehydgruppe kein H-Atom trägt. Geeignet sind insbesondere aromatische Aldehydverbindungen. Die Arylgruppe erfindungsgemäß eingesetzter aromatischer Aldehydverbindungen II kann einen oder mehrere, z.B. 1, 2, 3, 4 oder 5, unabhängig voneinander unter R", das dieselben Bedeutungen wie oben für R' definiert besitzt, ausgewählte Substituenten aufweisen.

Des Weiteren sind als nicht-enolisierbare Aldehyde II Verbindungen geeignet, deren Aldehydgruppe an einen unter
- C₁-C₁₀-Alkyl, das gegebenenfalls einen oder mehrere von Alkyl .verschiedene Substituenten, die unter Fluor, Chlor, Brom, Iod, C₁-C₁₀-Alkoxy oder C₃-C₁₀-Cycloalkyl ausgewählt sein können, aufweist;
- C₃-C₁₀-Cycloalkyl, das gegebenenfalls einen oder mehrere von Alkyl verschiedene Substituenten, die unter Fluor, Chlor, Brom, Iod oder C₁-C₁₀-Alkoxy ausgewählt sein können, aufweist;
- nichtaromatisches Heterocyclyl, das gegebenenfalls durch 1, 2, 3 oder 4 Reste R', wie oben definiert, substituiert ist; oder
- Heteroaryl, das gegebenenfalls durch 1, 2, 3 oder 4 Reste R', wie oben definiert, substituiert ist;
ausgewählten Rest gebunden ist.

Hier und im Folgenden steht nichtaromatisches Heterocyclyl für einen nichtaromatischen, heterocyclischen Rest mit in der Regel 3 bis 10, insbesondere 3 bis 6 und speziell 5 bis 6 Ringatomen, wobei üblicherweise 1, 2 oder 3 der Ringatome Heteroatome sind, die unter Sauerstoff, Schwefel und Stickstoff ausgewählt sind. Beispiele hierfür sind: Tetrahydrofuran-2-yl, Tetrahydrofuran-3-yl, Tetrahydrothiophen-2-yl, Tetrahydrothiophen-3-yl, Pyrrolidin-1-yl, Pyrrolidin-2-yl, Pyrrolidin-3-yl, Pyrrolidin-5-yl, Piperidin-1-yl, Piperidin-2-yl, Piperidin-3-yl, Piperidin-4-yl, Morpholin-2-yl, Morpholin-3-yl, Morpholin-4-yl, Hexahydropyridazin-1-yl, Hexahydropyridazin-3-yl, Hexahydropyridazin-4-yl, Piperazin-1-yl, Piperazin-2-yl, Piperazin-3-yl, Hexahydroazepin-1-yl, Hexahydroazepin-2-yl, Hexahydroazepin-3-yl, Hexahydroazepin-4-yl, Hexahydro-1,4-diazepin-1-yl, Hexahydro-1,4-diazepin-2-yl, Dihydrofuran-2-yl, 1,2-Oxazolin-3-yl, 1,2-Oxazolin-5-yl, 1,3-Oxazolin-2-yl.

Hier und im Folgenden steht aromatisches Heterocyclyl für einen ein- oder zweikernigen aromatischen, heterocyclischen Rest mit in der Regel 5 bis 10, insbesondere 3 bis 9 und speziell 5 bis 6 Ringatomen, wobei üblicherweise 1, 2 oder 3 der Ringatome Heteroatome sind, die unter Sauerstoff, Schwefel und Stickstoff ausgewählt sind. Beispiele hierfür sind: Furyl wie 2-Furyl und 3-Furyl, Thienyl wie 2-Thienyl und 3-Thienyl, Pyrrolyl wie 2-Pyrrolyl und 3-Pyrrolyl, Pyrazolyl wie 3-Pyrazolyl, 4-Pyrazolyl und 5-Pyrazolyl, Oxazolyl wie 2-Oxazolyl, 4-Oxazolyl und 5-Oxazolyl, Thiazolyl wie 2-Thiazolyl, 4-Thiazolyl und 5-Thiazolyl, Imidazolyl wie 2-Imidazolyl und 4-Imidazolyl, Oxadiazolyl wie 1,2,4-Oxadiazol-3-yl, 1,2,4-Oxadiazol-5-yl und 1,3,4-Oxadiazol-2-yl, Thiadiazolyl wie 1,2,4-Thiadiazol-3-yl, 1,2,4-Thiadiazol-5-yl und 1,3,4-Thiadiazol-2-yl, Triazolyl wie 1,2,4-Triazol-1-yl, 1,2,4-Triazol-3-yl und 1,2,4-Triazol-4-yl, Pyridinyl wie 2-Pyridinyl, 3-Pyridinyl und 4-Pyridinyl, Pyridazinyl wie 3-Pyridazinyl und 4-Pyridazinyl, Pyrimidinyl wie 2-Pyrimidinyl, 4-Pyrimidinyl und 5-Pyrimidinyl, des Weiteren 2-Pyrazinyl, 1,3,5-Triazin-2-yl und 1,2,4-Triazin-3-yl, insbesondere Pyridyl, Furanyl und Thienyl.

Bevorzugt verwendet man als nicht-enolisierbare Aldehydverbindung II eine Benzaldehyd-Verbindung, die gegebenenfalls am Phenylring einen oder mehrere, z.B. 1, 2, 3, 4 oder 5, unabhängig voneinander unter R", das dieselben Bedeutungen wie oben für R' definiert besitzt, ausgewählte Substituenten aufweist.

Derartige Verbindungen lassen sich durch die folgende Formel IIa beschreiben 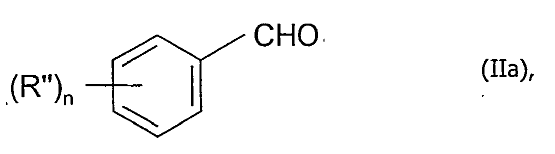 worin R" für n > 1 gleich oder verschieden sein kann und die zuvor genannten Bedeutungen aufweist; n für 0, 1, 2, 3, 4 oder 5, insbesondere für 0, 1 oder 2 und speziell für 1 steht. R" ist insbesondere ausgewählt unter Halogen.

In einer besonders bevorzugten Ausführungsform steht in der obigen Formel IIa R" für Halogen und n für 1. Insbesondere verwendet man als nicht-enolisierbare Aldehydverbindung II Benzaldehyd, der in 2-Position einen unter Fluor, Chlor, Brom oder Iod ausgewählten Substituenten aufweist. Speziell wird 2-Chlorbenzaldehyd eingesetzt.

In einer ganz besonders bevorzugten Ausführungsform setzt man als 2-Arylacetaldehyd 1 4-Fluorphenylacetaldehyd und als nicht-enolisierbare Aldehydverbindung II 2-Chlorbenzaldehyd ein.

Die Umsetzung nach dem erfindungsgemäßen Verfahren erfolgt vorteilhafterweise in einem Gemisch aus wenigstens einem mit Wasser mischbaren organischen Lösungsmittel und Wasser, in dem die Edukte, d. h. der 2-Arylacetaldehyd I und die nicht-enolisierbare Aldehydverbindung II, löslich sind. In einigen Fällen, wenn die nicht-enolisierbare Aldehydverbindung II nicht oder nur begrenzt löslich ist, kann es ausreichend sein, dass sie mit dem Lösungsmittelgemisch eine Suspension oder Emulsion bildet.

Das Lösungsmittelgemisch umfasst wenigstens ein mit Wasser mischbares organisches Lösungsmittel und Wasser in dem oben angegebenen Volumenverhältnis. Dabei wird die Menge des Wasseranteils geeigneterweise so eingestellt, dass die gebildeten Reaktionsprodukte eine möglichst geringe Löslichkeit in dem Lösungsmittelgemisch aufweisen (d. h. darin weitgehend unlöslich sind), während die Edukte eine zur Durchführung der Umsetzung ausreichende Löslichkeit in dem Lösungsmittelgemisch aufweisen. Der geeignete Wasseranteil kann vom Fachmann durch Routineversuche zur Löslichkeit ermittelt werden.

Die Auswahl des organischen Lösungsmittels und der Wassermenge zur Verwendung im erfindungsgemäßen Lösungsmittelgemisch erfolgt vorteilhafterweise derart, dass das als Reaktionsprodukt gebildete und gewünschte *cis*-Isomer unter den Reaktionsbedingungen als Feststoff.aus dem Lösungsmittelgemisch auskristallisiert.

Die Gesamtmenge der Lösungsmittel liegt im Allgemeinen zwischen 20 bis 10000 ml/mol, bevorzugt zwischen 50 bis 2000 ml/mol, besonders bevorzugt wischen 100 bis 1000 ml/mol und ganz besonders bevorzugt zwischen 300 bis 700 ml/mol, jeweils bezogen auf die nicht-enolisierbare Aldehydverbindung II.

Unter einem mit Wasser mischbaren organischen Lösungsmittel versteht man ein Lösungsmittel, das in der einzusetzenden Menge bei Raumtemperatur und Normaldruck mit Wasser eine homogene Mischung bildet. Bevorzugt sind organische Lösungsmittel, die mit Wasser unbegrenzt mischbar sind.

Das zur Verwendung im erfindungsgemäßen Lösungsmittelgemisch geeignete mit Wasser mischbare organische Lösungsmittel umfasst im Allgemeinen Alkohole, z.B. C₁-C₄-Alkanole, Diole, z.B. Ethylenglykol, und Polyole; Ether, wie etwa C₁C₂-Dialkylether, z.B. Dimethylether und Diethylether; Etheralkohole, z.B. (C₁-C₄-Alkyl)glykole und Diethylenglykol; Nitrile, wie etwa C₁-C₄-Alkylnitrile, z.B. Methannitril, Acetonitril und Propionitril; und/oder C₁-C₂-Alkylester der Ameisensäure, Essigsäure oder Propionsäure, z.B. Methyl- und Ethylformiat, -acetat und -propionat; ferner Dimethylsulfoxid und/oder Dimethylformamid; sowie Mischungen der vorgenannten Lösungsmittel.

Bevorzugt ist das organische Lösungsmittel ausgewählt unter C₁-C₄-Alkanolen, wie etwa Methanol, Ethanol, n-Propanol, iso-Propanol, n-Butanol, tert-Butanol; und/oder (C₁-C₄-Alkyl)glykolen, bevorzugt Mono(C₁-C₄-alkyl)glykolen, wie etwa Methoxyethanol, Ethoxyethanol, 2-Methoxypropanol und 2-Ethoxypropanol.

In einer besonders bevorzugten Ausführungsform der Erfindung ist das organische Lösungsmittel unter Methanol, Ethanol, n-Propanol, iso-Propanol, n-Butanol, iso-Butanol, tert-Butanol und/oder Methoxyethanol ausgewählt.

Zum Einsatz in der vorliegenden Erfindung geeignete Basen umfassen Alkali- und Erdalkalihydroxide, wie etwa Natrium-, Kalium-, Lithium-, Barium- und Calciumhydroxid; Alkali- und Erdalkalialkoholate, wie etwa Natrium- und Kaliummethanolat, -ethanolat, - propanolat, -iso-propanolat, -n-butanolat, -iso-butanolat, -tert-butanolat und cyclohexanolat; Alkali- und Erdalkaliphenolate, wie etwa Natrium- und Kaliumphenolat; Alkali- und Erdalkalicarboxylate, wie etwa Natrium-, Kalium-, Lithium- und Calciumcarbonat; sowie basische Salze, z. B. der Phosphorsäure oder Borsäure; ferner primäre und sekundäre Amine, wie etwa Piperidin, Pyrrolidin und Diisopropylamin. Bevorzugt werden Alkalimetallhydroxide, wie etwa NaOH oder KOH, besonders bevorzugt NaOH, eingesetzt.

Die Base .wird in der Regel in einer Menge von 0,5 bis 30 mol-%, bevorzugt 0,5 bis 20 mol-%, besonders bevorzugt 1 bis 15 mol-% und ganz besonders bevorzugt 2 bis 12 mol-%, bezogen auf die nicht-enolisierbare Aldehydverbindung II, verwendet.

Es hat sich als vorteilhaft erwiesen, die Umsetzung in Gegenwart eines aciden Cokatalysators durchzuführen. Besonders geeignet als acide Cokatalysatoren sind NH-acide und OH-acide Verbindungen mit einem pKs-Wert in Dimethytsulfoxid von maximal 25. Die Bestimmung des pKs-Wertes erfolgt nach dem Fachmann bekannten Methoden, wie z.B. in F.G. Bordwell, Accounts of Chem. Research 21 (1988), 456 beschrieben.

Insbesondere sind NH-acide Verbindungen mit einem pKs-Wert in Dimethylsulfoxid im Bereich von 10 bis 25 geeignet. Beispiele hierfür sind Sulfonamide, wie Benzolsulfonamid, Methansulfonamid oder Toluolsulfonamid; Imide, wie Phthalimid und Succinimid; Triazole, wie 1,2,4-Triazol; Uracil und Hydantoin. Bevorzugte NH-acide Cokatalysatoren sind Benzolsulfonamid, Methansulfonamid und Triazole, insbesondere 1,2,4-Triazol.

Als acide Cokatalysatoren geeignete OH-acide Verbindungen umfassen anorganische Säuren, wie Phosphorsäure, deren saure Salze wie Dihydrogenphosphate, und Borsäure; Carbonsäuren, beispielsweise aliphatische C₁-C₄-Carbon-, -dicarbon- und -hydroxycarbonsäuren, wie Ameisensäure, Essigsäure, Propionsäure, Buttersäure, Isobuttersäure, Oxalsäure, Malonsäure, Bernsteinsäure und Milchsäure, und aromatische Carbon- und Dicarbonsäuren, wie Benzoesäure, o-Phthalsäure, m-Phthalsäure und p-Phthalsäure; Phenole, wie p-Kresol und p-Chlorphenol; und Oxime, wie Ethanaloxim und Propanonoxim. Bevorzugte OH-acide Cokatalysatoren sind Benzoesäure, Ameisensäure und Borsäure.

Insbesondere verwendet man als acide Cokatalysatoren Benzolsulfonamid, Methansulfonamid, 1,2,4-Triazol, Benzoesäure, Ameisensäure oder Borsäure.

Der acide Cokatalysator wird in der Regel in einer Menge von 0,5 bis 500 mol-%, bevorzugt 1 bis 100 mol-%, besonders bevorzugt 5 bis 80 mol-% und ganz besonders bevorzugt 10 bis 50 mol-%, bezüglich der Base, eingesetzt.

Die Verwendung acider Cokatalysatoren im erfindungsgemäßen Verfahren führt dazu, dass die Bildung von Nebenprodukten, wie Triphenylpyranderivate, weitgehend oder vollständig vermieden wird.

Zur Umsetzung des 2-Arylacetaldehyds I mit der nicht-enolisierbaren Aldehydverbindung II ist es vorteilhaft, den 2-Arylacetaldehyd I, gegebenenfalls als Lösung oder Suspension, zu einer Lösung oder Suspension der nicht-enolisierbaren Aldehydverbindung II zu geben.

Die Lösungen/Suspensionen der Aldehyde I und II können.jeweils einen Teil oder die Gesamtmenge an organischem Lösungsmittel und/oder jeweils einen Teil oder die Gesamtmenge des bei der Umsetzung eingesetzten Wassers enthalten. Üblicherweise wird man so viel organisches Lösungsmittel bzw. Wasser einsetzen, dass die Verbindungen I bzw. II gelöst oder in einer Weise suspendiert sind; die eine leichte Handhabung der Lösungen/Suspensionen erlauben, insbesondere eine gute Dosierbarkeit und ein gutes Durchmischen gewährleisten. Diese Mengen können vom Fachmann in Routineversuchen zur Löslichkeit ermittelt werden. Im Übrigen ist es für die Durchführung des erfindungsgemäßen Verfahrens von untergeordneter Bedeutung; in welchem Mengenverhältnis die Lösungsmittel bzw. das Wasser auf die Lösungen/Suspensionen der Aldehyde I und II verteilt sind. Vorzugsweise wird jedoch die Lösung/Suspension des Aldehyds II Lösungsmittel und Wasser in dem oben angegebenen Mengenverhältnis enthalten.

In einer bevorzugten Ausführungsform legt man einen Teil der nicht-enolisierbaren Aldehydverbindung II, üblicherweise wenigstens 50%, insbesondere wenigstens 80% und besonders bevorzugt die Gesamtmenge vor. Vorzugsweise legt man den gewünschten Anteil an Aldehydverbindung II in einem Teil, z.B. wenigstens 50% und insbesondere in wenigstens 80% oder speziell in der Gesamtmenge an Lösungsmittel/Wasser-Gemisch vor. Dabei kann eine Lösung oder Suspension der nicht-enolisierbaren Aldehydverbindung II resultieren. Es kann auch von Vorteil sein, einen Teil des organischen Lösungsmittels oder Lösungsmittel/Wasser-Gemischs mit der Aldehydverbindung I der Reaktion zuzuführen.

Zur Vermeidung örtlich hoher Konzentrationen an 2-Arylacetaldehyd I hat es sich als vorteilhaft erwiesen, eine lineare Dosierung des 2-Arylacetaldehyds I bzw. dessen Lösung bei der Zugabe zur Lösung oder Suspension der nicht-enolisierbaren Aldehydverbindung II vorzunehmen. Unter linearer Dosierung versteht man die kontinuierliche Zugabe über einen längeren Zeitraum von in der Regel wenigstens 0,5 h und insbesondere wenigstens 1 h. Insbesondere gibt man den 2-Arylacetaldehyd I bzw. dessen Lösung mit gleich bleibender oder abnehmender Geschwindigkeit über einen Zeitraum im Bereich von 0,1 h bis 25 h, bevorzugt 0,5 h bis 10 h, besonders bevorzugt 1 h bis 6 h kontinuierlich zu einer Lösung oder Suspension von II zu.

In einer bevorzugten Ausführungsform der Erfindung erfolgt die Zugabe zunächst mit gleich bleibender Zugabegeschwindigkeit und gegen Ende der Zugabedauer, z.B. nachdem bereits 60 bis 80 Vol.-% von I zugegeben wurden, mit einer, z.B. auf das 0,8-fache, 0,5-fache oder 0,1-fache der ursprünglichen Zugabegeschwindigkeit, verringerten Zugaberate.

In einer weiteren bevorzugten Ausführungsform umfasst die Lösung oder Suspension der nicht-enolisierbaren Aldehydverbindung II die Base sowie gegebenenfalls den aciden Cokatalysator.

Die Umsetzung des 2-Arylacetaldehyds I mit der nicht-enolisierbaren Aldehydverbindung II erfolgt in der Regel im Temperaturbereich zwischen 0 und 100 °C, bevorzugt zwischen 5 bis 50 °C, besonders bevorzugt zwischen 10 bis 40 °C und ganz besonders bevorzugt zwischen 20 bis 30 °C.

Des Weiteren kann die Bildung von Nebenprodukten dadurch minimiert werden, dass die nicht-enolisierbare Aldehydverbindung II im Vergleich zum 2-Arylacetaldehyd I im Überschuss eingesetzt wird. Das molare Verhältnis des 2-Arylacetaldehyds I zur nicht-enolisierbaren Aldehydverbindung II liegt in der Regel im Bereich von 1:1,05 bis 1:5, besonders bevorzugt von 1:1,1 bis 1:3 und ganz besonders bevorzugt von 1:1,12 bis 1:1,30.

Eine Isolierung des gewünschten Reaktionsprodukts mit *cis*-Konfiguration ist durch übliche Verfahren; die dem Fachmann bekannt sind, wie z. B. Fällung, Extraktion oder Destillation, möglich.

In einer bevorzugten Ausführungsform erfolgt die Isolierung des Reaktionsprodukts mit *cis*-Konfiguration durch Fällung derart, dass das gebildete *cis*-Isomer (Reaktionsprodukt) unter den Reaktionsbedingungen als Feststoff aus dem Lösungsmittelgemisch auskristallisiert.

Wurde die nicht-enolisierbare Aldehydverbindung II im Verhältnis zum 2-Arylacetaldehyd I im Überschuss eingesetzt, so kann die überschüssige nicht-enolisierbare Aldehydverbindung II vollständig oder teilweise aus der Reaktionsmischung zurückgewonnen werden. Hierzu sind übliche, dem Fachmann bekannte Verfahren, wie etwa Fällung, Extraktion oder Destillation, geeignet.

In einer bevorzugten Ausführungsform erfolgt die Rückgewinnung der im Überschuss eingesetzten nicht-enolisierbaren Aldehydverbindung II derart, dass, nachdem das gewünschte Reaktionsprodukt abgetrennt wurde, eine Auftrennung der Reaktionsmischung in die organische und die wässrige Phase vorgenommen wird, z. B. durch Wasserdampfdestillation der Reaktionsmischung und anschließende fraktionierte Destillation. Je nach den Eigenschaften der nicht-enolisierbaren Aldehydverbindung II, wie z. B. Löslichkeits- und Siedeverhalten, gewinnt man diese dabei als isolierte Fraktion oder als eine Mischung mit der organischen oder der wässrigen Phase. Wird eine Mischung erhalten, so kann die Abtrennung auf eine übliche, dem Fachmann bekannte Weise, z. B. durch Fällung, Extraktion oder Destillation, erfolgen.

Durch das erfindungsgemäße Verfahren erhält man die 2,3-*cis*-substituierten Arylpropenale in hohen Ausbeuten und sehr guten Reinheiten, sowohl bezogen auf die gewünschte *cis*-Konfiguration als auch im Hinblick auf die Bildung von unerwünschten Nebenprodukten.

### Beispiele

### Beispiel 1

187,5 g 2-Chlorbenzaldehyd (CBA, 1,334 mol) werden in einer Mischung von 300 g Methanol und 160 g Wasser gelöst. Man setzt der Mischung 22 ml 1,6 gew.-%ige wässrige Natronlauge zu und temperiert auf 25 °C. Anschließend werden unter starkem Rühren 148 g 4-Fluorphenylacetaldehyd (dieser 4-Fluorphenylacetaldehyd (FPA) enthält einen 99,2 gew.-%igen Anteil an para-Fluorphenylacetaldehyd (p-FPA), was 1,063 mol entspricht) in 2 h zugetropft. Man lässt 1 h bei 20 °C nachrühren. Das ausgefallene *cis*-2-(4-Fluorphenyl)-3-(2-chlorphenyl)propenal (DPP) wird bei Raumtemperatur abgesaugt und mit 2 x 100 ml einer Mischung von 300 g MeOH und 160 g Wasser gewaschen.

Nach dem Trocknen werden 266 g DPP mit einem Anteil von 98,9 % *cis*-DPP erhalten. Der Gehalt an substituiertem Triphenylpyran (TPP) beträgt 0,34 %. Der Festpunkt des getrockneten Produkts beträgt 92,1 °C; die Ausbeute an DPP 95,0 %.

### Beispiel 2

170 g CBA (1,209 mol) werden in einer Mischung von 300 g Methanol und 90 g Wasser gelöst. Man setzt der Mischung 28 ml 16 gew.-%ige wässrige Natronlauge (0,13 mol) und 5 g Benzolsulfamid (0,03 mol) zu und temperiert auf 25 °C. Anschließend werden unter starkem Rühren 148 g FPA (99,2 % p-FPA, 1,063 mol) in 2 h zugetropft. Man lässt 1 h bei 20 °C nachrühren und stellt anschließend mit ca. 13 ml 31,5 %iger HCl einen pH-Wert von 8 - 10 ein. Das ausgefallene DPP wird bei Raumtemperatur abgesaugt und mit 2 x 100 ml einer Mischung von 300 g MeOH und 160 g Wasser gewaschen. Nach dem Trocknen werden 265 g DPP mit einem Anteil von 99,3 % cis-DPP erhalten. TPP ist nicht nachweisbar. Der Festpunkt des getrockneten Produkts beträgt 92,3 °C; die Ausbeute an DPP 94,8 %.

### Beispiel 3

187,5 g CBA (1,334 mol) werden in einer Mischung von 300 g Methanol und 130 g Wasser gelöst. Man setzt der Mischung 28 ml 16 gew.-%ige wässrige Natronlauge (0,13 mol) und 4 g Benzolsulfamid (0,03 mol) zu und temperiert auf 25 °C. Anschließend werden unter starkem Rühren 148 g FPA (99,2 % p-FPA, 1,063 mol) in 2 h zugetropft. Man lässt 1 h bei 20 °C nachrühren. Das ausgefallene DPP wird bei Raumtemperatur abgesaugt und mit 2 x 100 ml einer Mischung von 300 g MeOH und 130 g Wasser gewaschen.

Nach dem Trocknen werden 268 g DPP mit einem Anteil von 99,4 % *cis*-DPP erhalten. TPP ist nicht nachweisbar. Der Festpunkt des getrockneten Produkts beträgt 92,5 °C; die Ausbeute an DPP 95,9 %.

### Beispiele 4 bis 8

Man arbeitet nach Beispiel 2, ersetzt aber das Benzolsulfamid durch folgende H-acide Verbindungen: 4 = Methansulfonamid, 5 = 1,2,4-Triazol, 6 = Benzoesäure, 7 = HCOOH, 8 = H₃BO₃, jeweils 30 mmol, und verwendet 35 ml 16 gew.-%ige NaOH-Lösung. Es wurden folgende Ergebnisse erhalten:

**Tabelle 1**

| Beispiel | Cokatalysator | Ausbeute/% | Gehalt *cis*-DPP/ FI.-% (HPLC) | Fp./°C |
|---|---|---|---|---|
| 4 | Methansulfonamid | 94,8 | 98,62 | 92,6 |
| 5 | 1,2,4-Triazol | 94,0 | 98,21 | 92,3 |
| 6 | Benzoesäure | 95,2 | 98,89 | 92,4 |
| 7 | Ameisensäure | 94,7 | 98,53 | 91,9 |
| 8 | Borsäure | 94,6 | 98,02 | 91,5 |

Substituiertes Triphenylpyran (TPP) ist in keinem Fall nachweisbar (HPLC).

### Beispiel 9

In einem 1,25 m³ Rührbehälter werden zu einer Lösung von 154 kg 2-Chlorbenzaldehyd in 420 I einer Mischung von MeOH und Wasser (mit einem Wassergehalt von 23,5 Gew.-%) bei 15 °C 15 I wässrige Natronlauge (Gehalt: 16 Gew.-%) zugegeben und anschließend bei 20 - 25 °C 136 kg 4-Fluorphenylacetaldehyd (FPA, para-Anteil 99,1 %, Anteil Nebenkomponenten < 0,1 %) im Verlauf von 5 h zudosiert. Dabei kommt es zur Ausfällung von DPP. Die Fällung kann durch eine Nachreaktionszeit von 1 h bei 20 °C vervollständigt werden.

Man erhält nach der Separation an einer Schälzentrifuge und Waschen mit etwa 400 I einer 3:1-MeOH/Wasser-Mischung 260 kg mit einer Restfeuchte von 6,6 %. Das getrocknete Produkt hat einen Gehalt an *cis*-DPP von 98,5 %. Die Ausbeute an *cis-*DPP beträgt 95,1 % bezogen auf p-FPA. Als weitere Produkte sind enthalten: trans-DPP: 0,8 %; TPP: 0,4 %; CBA: 0,2 %.

Aus der Reaktionsmischung wird durch Wasserdampfdestillation eine Mischung von MeOH, Wasser und CBA erhalten, die anschließend über eine Kolonne in Methanol und eine Mischung von Wasser und 2-Chlorbenzaldehyd getrennt wird. Chlorbenzaldehyd scheidet sich als Unterphase ab und kann wiederverwendet werden.

### Beispiele 10 - 13

Man arbeitet wie in Beispiel 1, jedoch mit 300 g der unten angegebenen organischen Lösungsmittel und setzt die nachstehenden Mengen Wasser hinzu. Es wurden folgende Ergebnisse erhalten:

**Tabelle 2**

| Beispiel | Lösungsmittel | Wasser/g | Ausbeute/% | Gehalt *cis*-DPP/ Fl.-% (HPLC) | Fp./°C |
|---|---|---|---|---|---|
| 10 | tert-Butanol | 150 | 66,2 | 99,49 | 95,4 |
| 11 | Ethanol | 100 | 88,2 | 97,80 | 91,5 |
| 12 | n-Propanol | 125 | 75,5 | 99,60 | 95,3 |
| 13 | Methylglykol | 100 | 81,9 | 99,73 | 93,8 |

### Beispiel 14: Vergleichsbeispiel (nach DE 37 22 886)

4,0 g NaOH werden in 250 ml MeOH gelöst und unter Kühlung mit 155 g CBA versetzt. Anschließend werden bei 20 - 30 °C innerhalb von 4 - 5 h 138 g FPA zugesetzt. Man lässt 5 h bei 20 °C nachrühren und arbeitet in folgender Weise auf:
a) Absaugen des ausgefallenen Produkts, Waschen mit MeOH/Wasser (2:1) und trocknen.
   Gesamtausbeute an DPP: 67,6 %; Gehalt an *cis*-DPP: 93,0% (GC). Der Festpunkt beträgt 88,4 °C.
b) Man stellt mit 10 %iger H₂SO₄ einen pH-Wert von 7 ein, nimmt die organischen Produkte in 600 ml Methyl-tert-butylether auf, wäscht zweimal mit 200 ml Wasser, trocknet mit Na₂SO₄ und bestimmt dann mittels HPLC die Menge und Zusammensetzung der erhaltenen Mischung.
   Zusammensetzung: DPP: 90,6 %; *cis*-DPP: 77,4 %; trans-DPP: 13,2 %; Nebenprodukte: 9,2 %; DPP-Ausbeute (berechnet, nicht isoliert): 235 g (90,1 %).

## Patentansprüche

1. Verfahren zur Herstellung von 2,3-*cis*-substituierten 2-Arylpropenalen durch Kondensation eines 2-Arylacetaldehyds I mit einer nicht-enolisierbaren Aldehydverbindung II in Gegenwart einer Base, **dadurch gekennzeichnet, dass** man die Umsetzung in einem Lösungsmittelgemisch durchführt, das wenigstens ein mit Wasser mischbares organisches Lösungsmittel und Wasser in einem Volumenverhältnis V_{Lösungsmittel} : V_{wasser} von 10:1 bis 0,5:1 umfasst, wobei man den 2-Arylacetaldehyd I und die nicht-enolisierbare Aldehydverbindung II in einem molaren Verhältnis von I:II im Bereich von 1:1,05 bis 1:5 einsetzt.

2. Verfahren nach Anspruch 1, **dadurch gekennzeichnet, dass** das organische Lösungsmittel unter C₁-C₄-Alkanolen oder/und Mono(C₁-C₄-alkyl)glykolen ausgewählt ist.

3. Verfahren nach einem der vorhergehenden Ansprüche, **dadurch gekennzeichnet, dass** man als Base Alkali- oder/und Erdalkalimetallhydroxide verwendet.

4. Verfahren nach einem der vorhergehenden Ansprüche, **dadurch gekennzeichnet, dass** man die Base in einer Menge von 0,5 bis 30 mol-%, bezogen auf die nicht-enolisierbare Aldehydverbindung II, verwendet.

5. Verfahren nach einem der vorhergehenden Ansprüche, **dadurch gekennzeichnet, dass** man die Umsetzung in Gegenwart eines aciden Cokatalysators durchführt.

6. Verfahren nach Anspruch 5, **dadurch gekennzeichnet, dass** man den aciden Cokatalysator in einer Menge von 5 bis 80 mol-%, bezogen auf die Base, verwendet.

7. Verfahren nach Anspruch 5 oder 6, **dadurch gekennzeichnet, dass** der acide Cokatalysator unter NH-aciden Verbindungen mit einem pKs-Wert in Dimethylsulfoxid im Bereich von 10 bis 25, Borsäure, Phosphorsäure, Carbonsäuren, Phenolen und Oximen ausgewählt ist.

8. Verfahren nach einem der vorhergehenden Ansprüche, **dadurch gekennzeichnet, dass** man den 2-Arylacetaldehyd I zu einer Lösung oder Suspension der nicht-enolisierbaren Aldehydverbindung II zugibt.

9. Verfahren nach einem der vorhergehenden Ansprüche, **dadurch gekennzeichnet, dass** man den 2-Arylacetaldehyd I und die nicht-enolisierbare Aldehydverbindung II in einem molaren Verhältnis von I:II im Bereich von 1:1,1 bis 1:3 einsetzt und, gegebenenfalls, überschüssige nicht-enolisierbare Aldehydverbindung II vollständig oder teilweise zurückgewinnt.

10. Verfahren nach einem der vorhergehenden Ansprüche, **dadurch gekennzeichnet, dass** man als 2-Arylacetaldehyd I Phenylacetaldehyd, der gegebenenfalls am Phenylring einen oder mehrere unter Fluor, Chlor, Brom oder Iod ausgewählte Substituenten aufweist, einsetzt.

11. Verfahren nach einem der vorhergehenden Ansprüche, **dadurch gekennzeichnet, dass** man als nicht-enolisierbare Aldehydverbindung II Benzaldehyd, der gegebenenfalls am Phenylring einen oder mehrere unter
- C₁-C₁₀-Alkyl, das gegebenenfalls einen oder mehrere von Alkyl verschiedene Substituenten, die unter Fluor, Chlor, Brom, Iod, C₁-C₁₀-Alkoxy oder C₃-C₁₀-Cycloalkyl ausgewählt sein können, aufweist;
- C₁-C₁₀-Alkoxy, das gegebenenfalls einen oder mehrere von Alkyl verschiedene Substituenten, die unter Fluor, Chlor, Brom, Iod, C₁-C₁₀-Alkoxy oder C₃-C₁₀-Cycloalkyl ausgewählt sein können, aufweist;
- C₃-C₁₀-Cycloalkyl, das gegebenenfalls einen oder mehrere von Alkyl verschiedene Substituenten, die unter Fluor, Chlor, Brom, Iod oder C₁-C₁₀-Alkoxy ausgewählt sein können, aufweist;
- Phenoxy, das gegebenenfalls durch 1,2,3,4 oder 5 unter C₁-C₄-Alkyl, C₁-C₄-Alkoxy, Fluor, Chlor, Brom oder Iod ausgewählte Gruppen substituiert ist;
- Halogen, das unter Fluor, Chlor, Brom oder Iod ausgewählt ist;
- C₁-C₁₀-Acylamino; und
- einer Nitrogruppe
ausgewählte Substituenten aufweist, verwendet.

12. Verfahren nach einem der vorhergehenden Ansprüche, **dadurch gekennzeichnet, dass** man als 2-Arylacetaldehyd I 4-Fluorphenylacetaldehyd und als nicht-enolisierbare Aldehydverbindung II 2-Chlorbenzaldehyd einsetzt.

## Claims

1. A process for preparing 2,3-cis-substituted 2-arylpropenals by condensing a 2-arylacetaldehyde I with a nonenolizable aldehyde compound II in the presence of a base, which comprises carrying out the reaction in a solvent mixture which comprises at least one water-miscible organic solvent and water in a volume Vₛₒₗᵥₑₙₜ : V_{water} ratio of from 10:1 to 0.5:1, the 2-arylacetaldehyde I and the nonenolizable aldehyde compound II being used in a I:II molar ratio in the range from 1:1.05 to 1:5.

2. The process according to claim 1, wherein the organic solvent is selected from C₁-C₄-alkanols or/and mono(C₁-C₄-alkyl) glycols.

3. The process according to either of the preceding claims, wherein the base used is alkali metal hydroxides or/and alkaline earth metal hydroxides.

4. The process according to any of the preceding claims, wherein the base is used in an amount of from 0.5 to 30 mol%, based on the nonenolizable aldehyde compound II.

5. The process according to any of the preceding claims, wherein the reaction is carried out in the presence of an acidic cocatalyst.

6. The process according to claim 5, wherein the acidic cocatalyst is used in an amount of from 5 to 80 mol%, based on the base.

7. The process according to claim 5 or 6, wherein the acidic cocatalyst is selected from NH-acidic compounds having a pKₐ value in dimethyl sulfoxide in the range from 10 to 25, boric acid, phosphoric acid, carboxylic acids, phenols and oximes.

8. The process according to any of the preceding claims, wherein the 2-arylacetaldehyde I is added to a solution or suspension of the nonenolizable aldehyde compound II.

9. The process according to any of the preceding claims, wherein the 2-arylacetaldehyde I and the nonenolizable aldehyde compound II are used in a I:II molar ratio in the range from 1:1.1 to 1:3 and, if appropriate, excess nonenolizable aldehyde compound II is fully or partly recovered.

10. The process according to any of the preceding claims, wherein the 2-arylacetaldehyde I used is phenylacetaldehyde which optionally has one or more substituents selected from fluorine, chlorine, bromine and iodine on the phenyl ring.

11. The process according to any of the preceding claims, wherein the nonenolizable aldehyde compound II used is benzaldehyde which optionally has one or more substituents on the phenyl ring which are selected from
- C₁-C₁₀-alkyl which optionally has one or more substituents other than alkyl which may be selected from fluorine, chlorine, bromine, iodine, C₁-C₁₀-alkoxy and C₃-C₁₀-cycloalkyl;
- C₁-C₁₀-alkoxy which optionally has one or more substituents other than alkyl which may be selected from fluorine, chlorine, bromine, iodine, C₁-C₁₀-alkoxy and C₃-C₁₀-cycloalkyl;
- C₃-C₁₀-cycloalkyl which optionally has one or more substituents other than alkyl which may be selected from fluorine, chlorine, bromine, iodine and C₁-C₁₀-alkoxy;
- phenoxy which is optionally substituted by 1,2,3,4 or 5 groups selected from C₁-C₄-alkyl , C₁-C₄-alkoxy, fluorine, chlorine, bromine and iodine;
- halogen which is selected from fluorine, chlorine, bromine and iodine;
- C₁-C₁₀-acylamino; and
- a nitro group.

12. The process according to any of the preceding claims, wherein the 2-arylacetaldehyde I used is 4-fluorophenylacetaldehyde and the nonenolizable aldehyde compound II used is 2-chlorobenzaldehyde.

## Revendications

1. Procédé de fabrication de 2-arylpropénals substitués en position 2,3-cis en effectuant la condensation d'un 2-arylacétaldéhyde I avec un composé d'aldéhyde non énolisable II, en présence d'une base, **caractérisé en ce que** l'on effectue la réaction dans un mélange de solvants, qui comprend au moins un solvant organique miscible à l'eau et de l'eau dans un rapport volumique Vₛₒₗᵥₐₙₜ : Vₑₐᵤ de 10:1 à 0,5:1, dans lequel on utilise le 2-arylacétaldéhyde I et le composé d'aldéhyde non énolisable II dans un rapport molaire I:II situé dans la plage de 1:1,05 à 1:5.

2. Procédé selon la revendication 1, **caractérisé en ce que** le solvant organique est choisi parmi des alcanols en C₁-C₄ et/ou des mono(alkyl en C₁-C₄)-glycols.

3. Procédé selon l'une quelconque des revendications précédentes, **caractérisé en ce que** l'on utilise, comme base, des hydroxydes de métaux alcalins et/ou alcalinoterreux.

4. Procédé selon l'une quelconque des revendications précédentes, **caractérisé en ce que** l'on utilise la base en quantité de 0,5 à 30 % en mole par rapport au composé d'aldéhyde non énolisable II.

5. Procédé selon l'une quelconque des revendications précédentes, **caractérisé en ce que** l'on effectue la réaction en présence d'un cocatalyseur acide.

6. Procédé selon la revendication 5, **caractérisé en ce que** l'on utilise le cocatalyseur acide en quantité de 5 à 80 % en mole par rapport à la base.

7. Procédé selon la revendication 5 ou 6, **caractérisé en ce que** le cocatalyseur acide est choisi parmi des composés à fonction NH acide, ayant une valeur de pKa dans le sulfoxyde de diméthyle dans la plage de 10 à 25, l'acide borique, l'acide phosphorique, des acides carboxyliques, des phénols et des oximes.

8. Procédé selon l'une quelconque des revendications précédentes, **caractérisé en ce que** l'on ajoute le 2-arylacétaldéhyde I à une solution ou à une suspension du composé d'aldéhyde non énolisable II.

9. Procédé selon l'une quelconque des revendications précédentes, **caractérisé en ce que** l'on utilise le 2-arylacétaldéhyde I et le composé d'aldéhyde non énolisable II dans un rapport molaire I:II situé dans la plage de 1:1,1 à 1:3 et, éventuellement, **en ce que** l'on récupère complètement ou partiellement la quantité excédentaire du composé d'aldéhyde non énolisable II.

10. Procédé selon l'une quelconque des revendications précédentes, **caractérisé en ce que** l'on utilise comme 2-arylacétaldéhyde I, le phénylacétaldéhyde, qui présente, éventuellement, sur le cycle phényle un ou plusieurs substituants choisis parmi le fluor, le chlore, le brome ou l'iode.

11. Procédé selon l'une quelconque des revendications précédentes, **caractérisé en ce que** l'on utilise comme composé d'aldéhyde non énolisable II, le benzaldéhyde, qui présente, éventuellement, sur le cycle phényle un ou plusieurs substituants choisis parmi :
- un alkyle en C₁-C₁₀ qui présente, éventuellement, un ou plusieurs substituants différents de l'alkyle et que l'on peut choisir parmi le fluor, le chlore, le brome, l'iode, un alcoxy en C₁-C₁₀ ou un cycloalkyle en C₃-C₁₀ ;
- un alcoxy en C₁-C₁₀ qui présente, éventuellement, un ou plusieurs substituants différents de l'alkyle et que l'on peut choisir parmi le fluor, le chlore, le brome, l'iode, un alcoxy en C₁-C₁₀ ou un cycloalkyle en C₃-C₁₀ ;
- un cycloalkyle en C₃-C₁₀ qui présente, éventuellement, un ou plusieurs substituants différents de l'alkyle et que l'on peut choisir parmi le fluor, le chlore, le brome, l'iode ou un alcoxy en C₁-C₁₀ ;
- un phénoxy qui est substitué, éventuellement, par 1, 2, 3, 4 ou 5 groupements choisis parmi un alkyle en C₁-C₄, un alcoxy en C₁-C₄, le fluor, le chlore, le brome ou l'iode ;
- un halogène qui est choisi parmi le fluor, le chlore, le brome ou l'iode ;
- un acylamino en C₁-C₁₀ ; et
- un groupement nitro.

12. Procédé selon l'une quelconque des revendications précédentes, **caractérisé en ce que** l'on utilise, comme 2-arylacétaldéhyde I, le 4-fluorophényl-acétaldéhyde et, comme composé d'aldéhyde non énolisable II, le 2-chlorobenzaldéhyde.
